# FASCICULE DE BREVET EUROPEEN

(11) **EP 0 697 458 B1**
(45) Date de publication et mention de la délivrance du brevet: **05.01.2000**
(21) Numéro de dépôt: 95401415.5
(22) Date de dépôt: 16.06.1995
(51) Int. Cl.: C11B 9/00, C07C 69/92

(54) **Procédé d'obtention de compositions parfumantes et de produits parfumés et produits ainsi obtenus**
Verfahren zur Herstellung von parfumierter Zusammensetzungen und Produkten; die so herstellen Produkte
Process for making perfuming compositions and products; products obtained thereby

(30) Priorité: 24.06.1994 FR 9407811
(43) Date de publication de la demande: 21.02.1996
(73) Titulaire: RHODIA CHIMIE, 92408 Courbevoie Cédex (FR)
(72) Inventeur: Storet, Isabelle, F-38300 Les Eparres (FR); Crochemore, Michel, F-69630 Chaponost (FR)
(74) Mandataire: Dutruc-Rosset, Marie-Claude

(56) Documents cités:
- FR-A- 2 016 701
- S.ARCTANDER 'Perfume and Flavor Chemicals' 1969 , S.ARCTANDER , MONTCLAIR,N.J. * 230:iso-amyl vanillate * * 3066:vanillic acid *
- JOURNAL OF FOOD SCIENCE, vol. 57, no. 4, CHICAGO US, pages 985 - 993,1019 R.MRIRANDA-LOPEZ ET AL. 'Odor analysis of Pinot Noir grapes of different maturities by a gas chromatography-olfactory technique(Osme)'
- HELVETICA CHIMICA ACTA, vol. 52, no. 1, BASEL CH, pages 24 - 32
- PATENT ABSTRACTS OF JAPAN vol. 006, no. 165 (C-121) 28 Août 1982 & JP-A-57 082 308 (TAKASAGO CORP) 22 Mai 1982

## Description

La présente invention a trait à un procédé d'obtention de compositions parfumantes et de produits parfumés et produits ainsi obtenus.

La présente invention vise en particulier leur emploi dans le domaine de la parfumerie. Lesdits composés possèdent des propriétés olfactives intéressantes et peuvent être employés, entre autres, pour la préparation de compositions parfumantes et de produits parfumés.

L'industrie de la parfumerie est constamment à la recherche de produits qui par l'originalité, le volume et la puissance de leur fragrance puissent conférer aux compositions dans lesquelles elles interviennent, un caractère tout à fait particulier.

Il a maintenant été trouvé que les esters de l'acide hydroxybenzoïque définis ci-après, présentaient des propriétés olfactives originales.

Il est à noter qu'il est impossible pour l'Homme du Métier de prévoir si un composé chimique donné possèdera ou non, une odeur intéréssante du point de vue olfactif susceptible d'être retenue dans le domaine de la parfumerie.

Plus spécifiquement, la présente invention a pour objet un procédé pour l'obtention de compositions parfumantes, de substances et produits parfumés destinés à la parfumerie caractérisé par le fait que l'on ajoute aux constituants usuels de ces compositions, substances et produits finis, une quantité efficace d'un ester méthylique de l'acide 4-hydroxy-3-méthoxybenzoïque et/ou de l'acide 4-hydroxy-3-éthoxybenzoïque.

La présente invention a encore pour objet des compositions parfumantes, substances et produits parfumés caractérisés par le fait qu'ils comprennent, à titre de principe actif ayant une influence sur l'odeur, une quantité efficace d'un ester méthylique de l'acide 4-hydroxy-3-méthoxybenzoïque et/ou de l'acide 4-hydroxy-3-éthoxybenzoïque.

L'invention réside donc dans une nouvelle utilisation desdits esters, comme ingrédient parfumant.

Dans l'exposé qui suit de l'invention, ils seront désignés "esters méthyliques des acides hydroxybenzoïques".

Les composés précités exhalent une forte odeur de vanille, épicée très intéressante et différente de celle de la vanilline.

Ce sont des produits qui peuvent être utilisés, comme ingrédients parfumants, dans les compositions parfumantes, substances et produits parfumés.

Par "compositions parfumantes", on désigne des mélanges de divers ingrédients tels que solvants, supports solides ou liquides, fixateurs, composés odorants divers, etc..., dans lesquels sont incorporés les esters méthyliques des acides hydroxybenzoïques, lesquels sont utilisés pour procurer à divers types de produits finis, la fragrance recherchée.

Les bases pour parfum constituent des exemples préférés de compositions parfumantes dans lesquelles les esters méthyliques des acides hydroxybenzoïques peuvent être avantageusement utilisés.

Les eaux de toilettes, les lotions après rasage, les parfums, les savons, les gels de bain ou de douche ou les produits déodorants ou antiperspirants, qu'ils soient sous forme de sticks ou de lotions constituent des exemples de substances ou de produits finis dans lesquels les esters méthyliques des acides hydroxybenzoïques apportent leur note originale.

Ils peuvent intervenir aussi dans les shampooings et dans les produits capillaires de tout type.

Ils peuvent aussi parfumer les talcs ou poudres de toute nature.

Ils peuvent également convenir pour les désodorisants d'air ambiant ou tout produit d'entretien.

Un autre exemple de compositions dans lesquelles lesdits composés peuvent être introduits de façon avantageuse, est représenté par les compositions détergentes usuelles. Ces compositions comprennent généralement un ou plusieurs des ingrédients suivants : agents tensio-actifs anioniques, cationiques ou amphotères, agents de blanchiment, azurants optiques, charges diverses, agents anti-redéposition. La nature de ces divers composants n'est pas critique et les esters méthyliques des acides hydroxybenzoïques peuvent être ajoutés à tout type de composition détergente. Ils peuvent être introduits dans les adoucissants pour textiles, sous forme liquide ou dans les compositions déposées sur support, le plus souvent un non-tissé, destinées à être employées dans les sèche-linges.

La teneur des compositions selon l'invention en ester méthylique des acides hydroxybenzoïques exprimée en pourcentage en poids dans la composition considérée dépend de la nature de ladite composition (base pour parfum ou eau de toilette par exemple) et de la puissance et de la nature de l'effet recherché au niveau produit final. Il va de soi que dans une base pour parfum la teneur en ester méthylique des acides hydroxybenzoïques peut être très importante, par exemple supérieure à 50 % en poids et peut atteindre 90 % en poids tandis que dans un parfum, une eau de toilette ou une lotion après rasage, cette teneur pourra être très inférieure à 50 % en poids.

La teneur en ester méthylique des acides hydroxybenzoïques peut être dans les compositions détergentes, notamment ménagères ou dans des savons, de l'ordre de 1 à 2%.

Il peut également intervenir dans les shampooings parfumés à raison de 0,5 à 2 % ou pour parfumer tout produit capillaire.

Ainsi la limite inférieure de la teneur en ester méthylique des acides hydroxybenzoïques peut être celle qui provoque une modification perceptible à l'odorat de la fragrance ou de la note du produit fini. Dans certains cas, cette teneur minimale peut être de l'ordre de 0,01 % en poids. On peut évidemment faire appel à des teneurs non comprises dans les limites des teneurs indiquées ci-avant sans pour autant sortir du cadre de la présente invention.

Une des voie d'accès aux esters méthyliques des acides hydroxybenzoïques consistent à faire réagir l'acide 4-hydroxy-3-méthoxybenzoïque et/ou de l'acide 4-hydroxy-3-éthoxybenzoïque et le méthanol, en présence d'un catalyseur acide.

On trouve dans le commerce les acides de départ.

D'une manière préférentielle, on choisit de faire appel à un procédé d'estérification directe.

Les différentes réactions sont conduites en présence d'un catalyseur classique de type acide. On peut citer notamment, l'acide sulfurique, l'acide p-toluène sulfonique, les titanates d'alcoyle, de préférence le titanate d'isopropyle ou de n-butyle, l'oxyde d'antimoine.

La quantité des réactifs en présence est déterminée de telle sorte que le méthanol soit généralement en excès par rapport à l'acide hydroxybenzoïque. L'excès varie largement, de préférence de 50 à 500 % par rapport à la quantité stoechiométrique. Il est choisi plus préférentiellement entre 100 et 200 % de la quantité stoechiométrique.

La quantité de catalyseur utilisé, exprimée par rapport au poids de l'acide hydroxybenzoïque, est choisie avantageusement entre 1 et 5 %.

La température de la réaction est choisie de manière qu'elle soit suffisante pour permettre l'accomplissement de la réaction.

La température de la réaction est choisie de préférence entre 50 et 150°C.

La réaction est conduite avantageusement sous pression atmosphérique.

On conduit la réaction de préférence sous atmosphère d'un gaz inerte qui peut être l'azote ou un gaz rare, de préference l'argon.

D'un point de vue pratique, le procédé selon l'invention est simple à mettre en oeuvre.

Les différents réactifs peuvent être introduits dans n'importe quel ordre. D'une manière préférée, on choisit l'ordre des réactifs suivants : on introduit l'acide hydroxybenzoïque et le méthanol, puis le catalyseur acide.

On porte le milieu réactionnel à la température désirée, tout en maintenant le milieu réactionnel sous agitation.

Au cours de la réaction, il y a formation d'eau dans le milieu réactionnel. Une variante préférée de l'invention consiste à l'éliminer du milieu réactionnel, au fur et à mesure de leur formation, par tout moyen connu, notamment par distillation azéotropique (toluène, cumène ou pseudocumène).

En fin de réaction, on obtient l'ester méthylique de l'acide hydroxybenzoïque souhaité, le méthanol en excès et le catalyseur.

On peut récupérer l'ester méthylique de l'acide hydroxybenzoïque formé à partir du milieu réactionnel, par tout moyen approprié.

Ainsi, on peut notamment faire un lavage à l'eau suivie d'une neutralisation à l'aide d'une base.

La quantité de base, de préférence la soude, le carbonate ou le bicarbonate de sodium est telle que le pH soit compris entre 6 et 8.

On sépare la phase organique que l'on fractionne par distillation. On recueille le plus souvent, d'abord l'excès de méthanol puis l'ester méthylique des acides hydroxybenzoïques.

On donne ci-après des exemples de réalisation de l'invention.

### Exemple 1

1 - Dans cet exemple, on réalise la préparation de l'hydroxy-4 méthoxy-3 benzoate de méthyle dénommé vanillate de méthyle.
   Dans un réacteur équipé d'un agitation magnétique, d'un réfrigérant et d'un thermomètre, on additionne 50 g d'acide hydroxybenzoïque à 250 ml de méthanol. Par l'intermédiaire d'une ampoule à brome, on introduit goutte à goutte, 40 ml d'acide sulfurique concentré à 96 % ; on refroidit s'il y a ébullition de l'alcool.
   On chauffe 2 heures à reflux en agitant.
   Puis on refroidit à température ambiante, on coule sur environ 100 ml d'eau glacée, et on évapore l'alcool sous pression réduite (200 mm de mercure = 2,6.10⁴ Pa).
   On procède à trois extractions de la phase aqueuse par de l'éther éthylique.
   Les phases organiques combinées sont lavées par une solution saturée d'hydrogénocarbonate de sodium jusqu'à pH neutre, puis lavées une fois avec de l'eau de façon à éliminer les sels.
   On sèche la phase organique sur sulfate de magnésium et on évapore sous pression réduite (200 mm de mercure = 2,6.10⁴ Pa) pour obtenir l'ester brut.
   L'ester méthylique est distillé à 118°C, sous pression réduite de 2 mm de mercure (266 Pa) , puis recristallisé dans l'éther de pétrole (fraction 80-120°C), pour fournir le vanillate de méthyle d'une pureté supérieure à 97 % avec un rendement de 82 %.
2 - L'odeur de vanillate de méthyle est une odeur de vanille, épicée, différente de la vanilline.

### Exemple 2

On donne ci-après un exemple de mis en oeuvre du vanillate de méthyle dans une composition parfumante sous forme liquide.
- 50 ‰: Aldéhyde C. 11 undécylénique (solution 10 %)
- 5 ‰: Aldéhyde C.12 laurique (solution 10 %)
- 5 ‰: Aldéhyde C.12 méthylnonylacétique (solution 10 %)
- 180 ‰: Bergamote synthétique
- 50 ‰: Aurantiol® (solution 50 % diéthylphtalate) [méthyl-N-3,7-diméthyl-7-hydroxyoctylidène antranilate]
- 40 ‰: Salicylate de benzyle
- 90 ‰: Jasmin synthétique
- 80 ‰: Hédione® [dihydrojasmonate de méthyle]
- 50 ‰: Lyral® [4(4-hydroxy-4-méthylpentyl)-3-cyclohexène-1-carboxaldéhyde]
- 15 ‰: Eugènol
- 5 ‰: Isoeugènol
- 80 ‰: Alcool phényléthylique
- 60 ‰: Méthylionone
- 80 ‰: Vertofix® [cédr-8-énylméthyl-cétone]
- 60 ‰: Patchouli Singapour "déférisé"
- 20 ‰: Coumarine
- 80 ‰: Vanillate de méthyle
- 50 ‰: Moskène® [4,6-dinitro-1,1,3,3,5-pentaméthylindane]

Le parfum obtenu est du type parfum épicé.

### Exemple 3

On a préparé l'ester méthylique de l'acide 4-hydroxy-3-éthoxybenzoïque. La description olfactive du produit obtenu est oeillet.

## Revendications

1. Procédé pour l'obtention de compositions parfumantes, de substances et produits parfumés destinés à la parfumerie caractérisé par le fait que l'on ajoute aux constituants usuels de ces compositions, substances et produits finis, une quantité efficace d'un ester méthylique de l'acide 4-hydroxy-3-méthoxybenzoïque et/ou de l'acide 4-hydroxy-3-éthoxybenzoïque.

2. Compositions parfumantes, substances et produits parfumés caractérisés par le fait qu'ils comprennent, à titre de principe actif ayant une influence sur l'odeur, une quantité efficace d'un ester méthylique de l'acide 4-hydroxy-3-méthoxybenzoïque et/ou de l'acide 4-hydroxy-3-éthoxybenzoïque.

3. Article parfumé selon la revendication 2 sous forme de parfum, d'eau de toilette, de lotions après rasage, de parfums, de savons, de gels de bain ou de douche, de produits déodorants ou antiperspirants, de shampooings ou tout produit capillaire, de talcs ou poudres de toute nature, de désodorisants d'air ambiant, de tout produit d'entretien ou de compositions détergentes, d'adoucissants pour textiles.

4. Utilisation comme ingrédient parfumant, de l'ester méthylique de l'acide 4-hydroxy-3-méthoxybenzoïque et/ou de l'acide 4-hydroxy-3-éthoxybenzoïque.

## Claims

1. A process for the production of perfuming compositions, and perfumed substances and perfumed products for use in the perfume industry, characterised in that an effective quantity of a methyl ester of 4-hydroxy-3-methoxybenzoic acid and/or of 4-hydroxy-3-ethoxybenzoic acid is added to the usual constituents of these compositions, substances and finished products.

2. Perfuming compositions, perfumed substances and perfumed products, characterised in that they comprise an effective quantity of a methyl ester of 4-hydroxy-3-methoxybenzoic acid and/or of 4-hydroxy-3-ethoxybenzoic acid as an active ingredient which influences the scent.

3. A perfumed article according to claim 2, in the form of a perfume, toilet water, after-shave lotion, soap, bath or shower gel, a deodorising or antiperspirant product, shampoo or any hair care product, any type of talc or powder, room deodoriser, any cleaning material or detergent composition, or a fabric softener.

4. The use of a methyl ester of 4-hydroxy-3-methoxybenzoic acid and/or of 4-hydroxy-3-ethoxybenzoic acid as a perfuming ingredient.

## Patentansprüche

1. Verfahren zur Herstellung von parfümierenden Zusammensetzungen sowie parfümierten Substanzen und Produkten der Kosmetikindustrie, dadurch gekennzeichnet, daß man den üblichen Komponenten dieser Zusammensetzungen, Substanzen und Endprodukte eine wirksame Menge eines Methylesters der 4-Hydroxy-3-methoxybenzoesäure und/oder der 4-Hydroxy-3-ethoxybenzoesäure zugibt.

2. Parfümierende Zusammensetzungen, Substanzen und Produkte, dadurch gekennzeichnet, daß sie als einen auf den Geruch Einfluß habenden Wirkstoff eine wirksame Menge eines Methylesters der 4-Hydroxy-3-methoxybenzoesäure und/oder der 4-Hydroxy-3-ethoxybenzoesäure enthalten.

3. Parfümierte Ware nach Anspruch 2 in Form von Parfüm, Eau de Toilette, Aftershave-Lotion, Seifen, Badezusätzen, Duschgel, Deodorants, Antiperspirants, Shampoos oder allen Haarprodukten, jeglicher Form von Talkum oder Pudern, Raumluftverbesserern, allen Pflegeprodukten oder Detergentien und Textilpflegeprodukten.

4. Verwendung des Methylesters der 4-Hydroxy-3-methoxybenzoesäure und/oder der 4-Hydroxy-3-ethoxylbenzoesäure als parfümierender Bestandteil.
